# EUROPEAN PATENT APPLICATION

(11) **EP 3 982 110 A1**
(43) Date of publication of application: **13.04.2022**
(21) Application number: 21200673.8
(22) Date of filing: 04.10.2021
(51) Int. Cl.: G01N 21/27, G01N 21/82, G01N 33/49, G01N 33/86, A61B 5/00

(54) **BLOOD SPECIMEN ANALYSIS METHOD, ANALYZER, AND ANALYSIS PROGRAM**

(30) Priority: 09.10.2020 JP 2020171206
(71) Applicant: SYSMEX CORPORATION, Kobe-shi Hyogo 651-0073 (JP)
(72) Inventor: Tabuchi, Yuka, Kobe-shi, Hyogo, 651-0073 (JP); Kimura, Konobu, Kobe-shi, Hyogo, 651-0073 (JP); Nishi, Keisuke, Kobe-shi, Hyogo, 651-0073 (JP); Kumano, Osamu, Kobe-shi, Hyogo, 651-0073 (JP)
(74) Representative: Hoffmann Eitle

(57) **Abstract**

Disclosed is an analysis method for a blood specimen, including: obtaining a data group including a plurality of data forming a blood coagulation curve or a differential curve thereof; inputting the data group into a deep learning algorithm; and outputting, on the basis of a result obtained from the deep learning algorithm, information regarding a cause of prolongation of blood coagulation time of the blood specimen.

## Description

### CROSS REFERENCE TO RELATED APPLICATIONS

This application claims priority from prior Japanese Patent Application No. 2020-171206, filed on October 9, 2020, entitled "BLOOD SPECIMEN ANALYSIS METHOD, ANALYZER, AND ANALYSIS PROGRAM", the entire content of which is incorporated herein by reference.

### FIELD OF THE INVENTION

The present specification discloses a blood specimen analysis method, an analyzer, and an analysis program.

### BACKGROUND

In a blood coagulation test, when the blood coagulation time has been prolonged, identification of the cause is performed by a medical worker. As a method for supporting identification of a cause of blood coagulation time prolongation, US Patent No. 6321164 discloses a method for predicting, by use of a neural network, coagulation factor deficiency, contamination of heparin into the specimen, and the presence of lupus anticoagulant. In US Patent No. 6321164, a PT or APTT time-dependent optical profile is obtained; and one or more predictor variables selected from a minimum value of a first derivative of the optical profile, a time index of the minimum value of the first derivative, a minimum value of a second derivative of the optical profile, a time index of the minimum value of the second derivative, a maximum value of the second derivative, a time index of the maximum value of the second derivative, the overall change of transmittance during reaction, a coagulation time, the slope of the optical profile before clot formation, and the slope of the optical profile after the clot formation, are used as input values to the neural network.

### SUMMARY OF THE INVENTION

In the method described in US Patent No. 6321164, the input values to the neural network are limited to specific predictor variables. Therefore, when changes in the predictor variables with respect to a plurality of prolongation causes are similar, prediction is difficult, and thus, improvement of prediction accuracy has been desired.

The present invention addresses providing a blood specimen analysis method, an analyzer, and an analysis program that can predict prolongation causes with higher accuracy than before.

An embodiment disclosed in the present specification relates to an analysis method for a blood specimen. The analysis method includes: obtaining a data group including a plurality of data forming a blood coagulation curve or a differential curve thereof; inputting the data group into a deep learning algorithm; and outputting, on the basis of a result obtained from the deep learning algorithm, information regarding a cause of prolongation of blood coagulation time of the blood specimen.

Another embodiment disclosed in the present specification relates to an analyzer (1) for a blood specimen. The analyzer (1) includes a measurement unit (2) and a controller (201). The measurement unit (2) is configured to prepare a measurement sample that contains the blood specimen and a coagulation time measurement reagent, and configured to output a plurality of pieces of detection information forming a blood coagulation curve, on the basis of the measurement sample. The controller (201) is configured to: obtain a data group including a plurality of data forming the blood coagulation curve or a differential curve thereof, on the basis of the plurality of pieces of detection information; input the data group into a deep learning algorithm; and output, on the basis of a result obtained from the deep learning algorithm, information regarding a cause of prolongation of blood coagulation time of the blood specimen.

Another embodiment disclosed in the present specification relates to an analysis program (202b) for a blood specimen. The analysis program (202b) is configured to cause, when executed by a computer, the computer to execute the steps of: obtaining a data group including a plurality of data forming a blood coagulation curve or a differential curve thereof; inputting the data group into a deep learning algorithm; and outputting, on the basis of a result obtained from the deep learning algorithm, information regarding a cause of prolongation of blood coagulation time of the blood specimen.

According to the present invention, it is possible to predict a prolongation cause with higher accuracy than before.

### BRIEF DESCRIPTION OF THE DRAWINGS

FIG. 1 shows an example of the appearance of an analyzer 1;
FIG. 2 shows an example of a hardware configuration of the analyzer 1;
FIG. 3 shows a configuration example of a light applicator 10;
FIG. 4 shows a configuration example of a detector 230;
FIG. 5 shows the flow of a measurement/analysis process performed by a controller 201 of the analyzer 1;
FIG. 6 shows the flow of a measurement process executed by the controller 201 on the basis of a measurement program 202a;
FIG. 7 shows the flow of an analysis process executed by the controller 201 on the basis of an analysis program 202b;
FIG. 8A shows an example of a blood coagulation curve;
FIG. 8B shows an example of a normalized coagulation curve;
FIG. 9A shows an example of a first-order differential coagulation curve;
FIG. 9B shows an example of a second-order differential coagulation curve;
FIG. 10 shows the outline of training of a deep learning algorithm;
FIG. 11 shows an example of analysis using the deep learning algorithm;
FIG. 12 shows a modification of a data group including a plurality of data;
FIG. 13A is an example in which probability indicated in a softmax form is outputted as a pie graph;
FIG. 13B is an example in which probability indicated in a binary form is outputted as a bar graph;
FIG. 14 shows an example of information stored in an additional test DB 202e;
FIG. 15A shows an example of the appearance of a training apparatus 5;
FIG. 15B shows an example of a hardware configuration of the training apparatus 5;
FIG. 16 shows an example of a training process executed by a controller 501 on the basis of a training program 502b;
FIG. 17 shows an example of a hardware configuration of a modification of the analyzer 1;
FIG. 18 shows the flow of a process executed on the basis of an analysis program 202b' by a controller 201 of the analyzer 1';
FIG. 19A is a histogram showing a prediction result according to a conventional method;
FIG. 19B is a histogram showing a prediction result according to the present embodiment;
FIG. 20A shows an ROC curve according to a conventional method;
FIG. 20B shows an ROC curve according to the present embodiment;
FIG. 21A shows an ROC curve of warfarin-administered blood specimens, which is a result of discrimination obtained by performing PT measurement on the warfarin-administered blood specimens;
FIG. 21B shows an ROC curve of DOACs-administered blood specimens, which is a result of discrimination obtained by performing PT measurement on the DOACs-administered blood specimens;
FIG. 21C shows an ROC curve of decreased liver function blood specimens, which is a result of discrimination obtained by performing PT measurement on the decreased liver function blood specimens;
FIG. 22A shows an ROC curve of FVIII inhibitor positive blood specimens, which is a result of discrimination obtained by performing APTT measurement on the FVIII inhibitor positive blood specimens;
FIG. 22B shows an ROC curve of FVIII deficient blood specimens, which is a result of discrimination obtained by performing APTT measurement on the FVIII deficient blood specimens;
FIG. 22C shows an ROC curve of LA positive blood specimens, which is a result of discrimination obtained by performing APTT measurement on the LA positive blood specimens;
FIG. 22D is an ROC curve of heparin-administered blood specimens, which is a result of discrimination obtained by performing APTT measurement on the heparin-administered blood specimens;
FIG. 22E is an ROC curve of DOACs-administered blood specimens, which is a result of discrimination obtained by performing APTT measurement on the DOACs-administered blood specimens;
FIG. 23A shows an ROC curve of FVIII inhibitor positive blood specimens, which is a result of discrimination obtained by performing PT measurement and APTT measurement on the FVIII inhibitor positive blood specimens;
FIG. 23B shows an ROC curve of LA positive blood specimens, which is a result of discrimination obtained by performing PT measurement and APTT measurement on the LA positive blood specimens;
FIG. 23C shows an ROC curve of decreased liver function blood specimens, which is a result of discrimination obtained by performing PT measurement and APTT measurement on the decreased liver function blood specimen;
FIG. 23D shows an ROC curve of heparin-administered blood specimens, which is a result of discrimination obtained by performing PT measurement and APTT measurement on the heparin-administered blood specimens; and
FIG. 23E shows an ROC curve of DOACs-administered blood specimens, which is a result of discrimination obtained by performing PT measurement and APTT measurement on the DOACs-administered blood specimens.

### DETAILED DESCRIPTION OF THE PREFERRED EMBODIMENTS

### 1. Analyzer

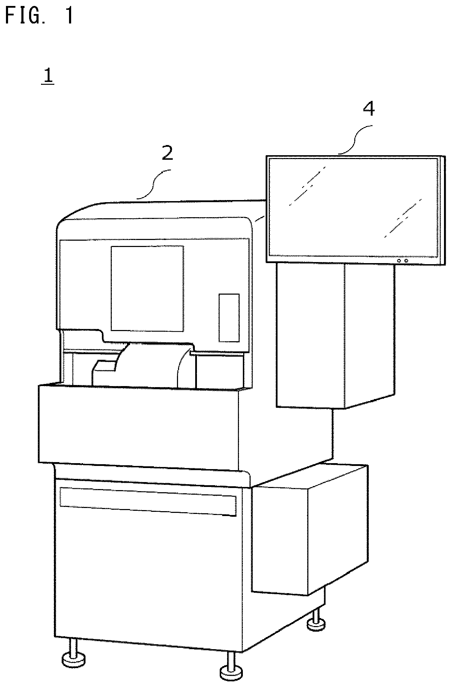

With reference to FIG. 1 to FIG. 14, an analyzer (hereinafter, simply referred to as an "analyzer 1") of the present embodiment is described.

### 1-1. Hardware configuration of analyzer

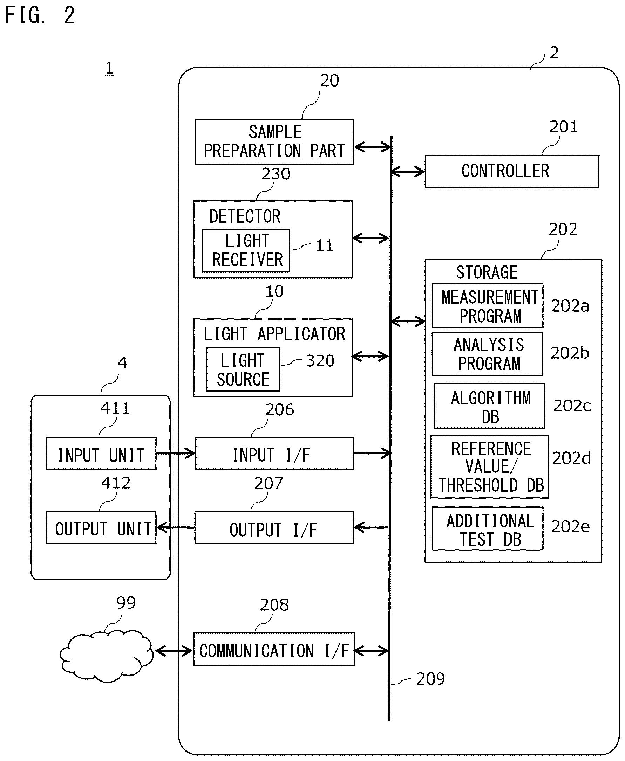

The analyzer 1 is an apparatus in which light is applied to a measurement sample prepared by adding a coagulation measurement reagent to a blood specimen, transmitted light of the light applied to the measurement sample is detected, and the blood specimen is analyzed on the basis of the detected light. FIG. 1 shows an example of the appearance of the analyzer 1 of the present embodiment. The analyzer 1 includes: a measurement unit 2 for obtaining detection information; and a display 4 to which data can be inputted in a touch panel manner. FIG. 2 shows an example of a hardware configuration of the analyzer 1.

The measurement unit 2 of the analyzer 1 includes a controller 201, a storage 202, a light applicator 10, a sample preparation part 20, a detector 230, an input interface (I/F) 206, an output interface (I/F) 207, a communication interface (I/F) 208, and a bus 209.

The controller 201 includes an arithmetic processing device such as a CPU (Central Processing Unit) or an FPGA (field-programmable gate array).

The storage 202 stores: a measurement program 202a for controlling measurement operation performed by the measurement unit 2; an analysis program 202b; an algorithm database (DB) 202c storing one or a plurality of deep learning algorithms 60; a reference value/threshold database (DB) 202d storing a reference value for a blood coagulation time of each blood coagulation parameter, and a threshold for a probability that a cause candidate for blood coagulation time prolongation is the cause of blood coagulation time prolongation; and an additional test database (DB) 202e storing information of additional tests.

The input interface 206 receives input information inputted by an operator through an input unit 411 of the display 4, and transmits the input information to the controller 201 or the storage 202.

The output interface 207 transmits, to an output unit 412 of the display 4, output information outputted by the controller 201.

The communication interface 208 communicably connects the measurement unit 2 to a network 99. The connection may be wired connection or wireless connection.

Signal transmission in the measurement unit 2 is performed via the bus 209.

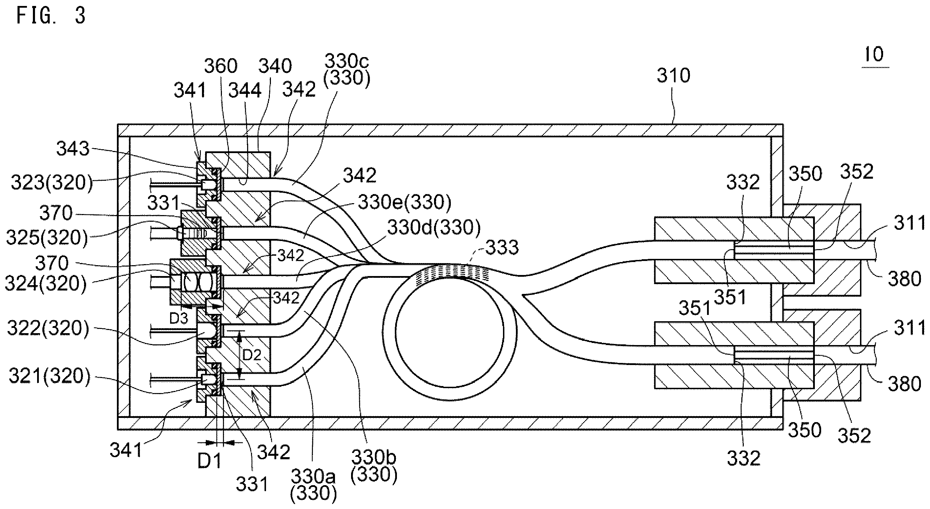

FIG. 3 shows a configuration example of the light applicator 10. In the configuration example in FIG. 3, the light applicator 10 includes: five light sources 320; five optical fiber parts 330 provided so as to correspond to the five light sources 320; and one holding member 340 for holding each light source 320 and a light entry end 331 of the corresponding optical fiber part 330. The light sources 320, the optical fiber parts 330, and the holding member 340 are housed in a housing 310 made of metal, for example.

Each of the five light sources 320 is implemented as an LED. In general, the life of an LED is several tens of times as long as that of a halogen lamp. Therefore, when compared with a configuration in which a wide-band light source such as a halogen lamp and a rotary filter are used, a light applicator 10 that is smaller and that has a longer life can be configured. In addition, since LEDs can be provided individually for respective wavelengths, emission spectra and emission intensities of the respective light sources 320 can be individually optimized.

The light sources 320 include a first light source 321, a second light source 322, and a third light source 323.

In the configuration example in FIG. 3, the first light source 321 is a light source, for blood coagulation time measurement, that generates light having a wavelength of about 660 nm as a first wavelength. The second light source 322 is a light source that generates light having a wavelength of about 405 nm as a second wavelength. The third light source 323 is a light source that generates light having a wavelength of about 800 nm as a third wavelength.

In the configuration example in FIG. 3, the plurality of light sources 320 further include a fourth light source 324 for generating light having a fourth wavelength different from the second wavelength. Similar to the second wavelength, the fourth wavelength is a wavelength selected from a range of not less than 300 nm and not greater than 380 nm. More preferably, light in a wavelength band of 320 nm to 360 nm can be used. In the configuration example in FIG. 3, the fourth wavelength is 340 nm, for example.

In the configuration example in FIG. 3, the plurality of light sources 320 further include a fifth light source 325 for generating light having a fifth wavelength different from the third wavelength. Similar to the third wavelength, the fifth wavelength is a wavelength selected from a range of not less than 550 nm and not greater than 590 nm. More preferably, light in a wavelength band of 560 nm to 580 nm can be used. In the configuration example in FIG. 3, the fifth wavelength is 575 nm, for example.

The optical fiber parts 330 are provided so as to correspond to the respective light sources 320. The five optical fiber parts 330 are implemented as optical fiber parts 330a, 330b, 330c, 330d, and 330e that are individually provided for the respective light sources 320 such that lights from the first light source 321, the second light source 322, the third light source 323, the fourth light source 324, and the fifth light source 325 enter from the respective light entry ends 331.

In the configuration example in FIG. 3, the plurality of optical fiber parts 330 each include a plurality of optical fibers 333. The plurality of optical fiber parts 330 are bundled so as to be mixed such that, at a light outputting end 332, a plurality of optical fibers 333 corresponding to each light source 320 are substantially uniformly distributed. Here, an "optical fiber" means an optical fiber element wire or optical fiber core wire that has one core. Each optical fiber part 330 is formed as a cable or a strand obtained by bundling a plurality of element wires. Due to this configuration, instead of individually applying, to a container 15, lights having respective wavelengths having separately entered the light entry ends 331 of the respective optical fiber parts 330, it is possible to cause the lights to be outputted from a common light outputting end 332. Therefore, the configuration for outputting lights having respective wavelengths can be simplified.

In addition, at the common light outputting end 332, light can be outputted in a state where the lights having the respective wavelengths are uniformly distributed. Therefore, even when lights having the respective wavelengths are outputted from the common light outputting end 332, biased distribution of lights of the respective wavelengths can be suppressed.

In the configuration example in FIG. 3, the five optical fiber parts 330 are twisted to be integrated at a position and the integrated body is provided with two light outputting ends 332. The two light outputting ends 332 are provided so as to respectively correspond to two detectors 230. The two light outputting ends 332 are respectively connected to two outlets 311 provided in the housing 310. Each light outputting end 332 includes a substantially equal number of optical fibers 333 of each optical fiber part 330. In addition, optical fibers 333 of each optical fiber part 330 are mixed so as to be substantially uniformly distributed at the end face of the light outputting end 332. The number of optical fibers 333 of each optical fiber part 330 is determined in accordance with the number of container setting parts 231 in the detectors 230 and 240. For example, when the number of container setting parts 231 is defined as N, and each optical fiber part 330 transmits a light amount corresponding to M optical fibers to one container setting part 231, each optical fiber part 330 includes N×M optical fibers 333. Each light outputting end 332 is formed so as to have (N×M)/2 optical fibers 333 out of the optical fiber parts 330.

In the configuration example in FIG. 3, the light applicator 10 further includes a uniformization member 350 that is disposed so as to be adjacent to each light outputting end 332 of the optical fiber part 330 and that is for uniformizing the distribution of intensity of light having entered from the light outputting end 332 side and for outputting the light. Here, each optical fiber 333 disposed at the light outputting end 332 outputs only one of the lights having the first wavelength to the fifth wavelength. That is, at the light outputting end 332, emission points for the respective wavelengths are disposed so as to be uniformly distributed. Thus, as a result of causing the outputted light to enter the uniformization member 350 to be made uniform, a state where the intensity distribution of the wavelengths is made uniform over the entirety of the face is established at a light outputting face 352 of the uniformization member 350. Accordingly, varied light intensities for the respective wavelengths can be effectively made uniform.

The uniformization member 350 is disposed at each of the two outlets 311 provided in the housing 310. As for each uniformization member 350, a light entry face 351 is opposed to the corresponding light outputting end 332 of the optical fiber part 330, and a light outputting face 352 is disposed on the exit side at the outlet 311. Accordingly, light of which the intensity distribution has been made uniform through the uniformization member 350 is outputted from each outlet 311. The uniformization member 350 is configured such that, for example, light having entered from the light entry face 351 is reflected multiple times to be outputted from the light outputting face 352.

In a case where the intensity distribution of lights having the respective wavelengths is sufficiently made uniform at the light outputting end 332 of the optical fiber part 330, the uniformization member 350 need not necessarily be provided.

The holding member 340 of the light applicator 10 holds the five light sources 320. Therefore, the five light sources 320 are supported by the common holding member 340. The holding member 340 is made of metal such as aluminum, for example, and is formed in a prism shape. In the configuration example in FIG. 3, a light source holder 341 and a light entry end holder 342 are respectively provided at one end portion and the other end portion of the holding member 340, and are connected to each other through a passage portion 344 formed as a through-hole penetrating the holding member 340.

The five light source holders 341 are disposed so as to be linearly arranged along a direction orthogonal to the outputting direction of the light of each light source 320. As for the light sources 320, the fourth light source 324 is disposed at the center, the fifth light source 325 and the second light source 322 are disposed on both sides of the fourth light source 324, and the first light source 321 and the third light source 323 are disposed on the outermost sides.

In the configuration example in FIG. 3, the plurality of light source holders 341 holding the respective light sources 320, and the plurality of light entry end holders 342 respectively holding the light entry ends 331 of the plurality of optical fiber parts 330 are disposed at positions where the plurality of light source holders 341 and the plurality of light entry end holders 342 face each other in a linear manner in the holding member 340. Accordingly, the optical axis of each light source 320 and the axis center of the corresponding optical fiber part 330 at the light entry end 331 can be easily matched with each other with high accuracy. Each light source holder 341 and the corresponding light entry end holder 342 are disposed at positions where the light source holder 341 and the light entry end holder 342 are opposed to each other on a substantially same axial line.

In the configuration example in FIG. 3, each light source holder 341 holds a light source 320 via a socket 343. The light source holder 341 includes a recess 345 connected to the passage portion 344, and the socket 343 is a tubular member fitted in the recess 345. The light source 320 is fixedly held inside the socket 343. The light entry end holder 342 is implemented as the other end portion of the passage portion 344 formed as a through-hole penetrating the holding member 340. Therefore, the light entry end holder 342 is a hole portion allowing the light entry end 331 to be inserted therein, and holds a predetermined range of length, including the light entry end 331, of the optical fiber part 330 inserted therein.

The light applicator 10 may be provided with a member for condensing the light from each light source 320 on the light entry end 331 of the optical fiber part 330, and a member for adjusting spectrum characteristics such as the center wavelength or the half-width of light entering the light entry end 331.

For example, the light applicator 10 further includes optical band-pass filters 360 that each allow only light in a predetermined wavelength band to be transmitted therethrough. Each optical band-pass filter 360 has a disk-like shape, and allows, out of the light applied to one surface thereof, only light in a predetermined wavelength band to be transmitted to the other surface. The holding member 340 holds each optical band-pass filter 360 at a position between a light source 320 and a light entry end 331 of a corresponding optical fiber part 330. Accordingly, the center wavelength or half-width of the light outputted from the light source 320 can be adjusted so as to have characteristics appropriate for measurement, to be caused to enter the light entry end 331. As a result, measurement accuracy is improved. Although there are cases where individual differences are present in the light sources 320 and the center wavelength and half-width are different, it is possible to absorb influence of the individual differences of each light source 320 by the optical band-pass filter 360, thereby ensuring a stable measurement result.

Here, an example in which LEDs are used as the light sources has been described. However, for example, a halogen lamp may be used as a light source, the light thereof may be split, by band-pass filters or the like, into lights having the first wavelength to the fifth wavelength, and the respective lights may be applied to the measurement sample.

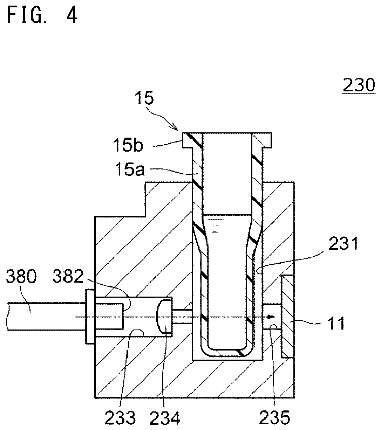

FIG. 4 shows a configuration example of the detector 230. The detector 230 includes a container setting part 231 as a hole portion extending in the up-down direction, and a light outputting end 382 of a light distribution member 380 is disposed in a hole 233 laterally extending from the container setting part 231. A condenser lens 234 is disposed inside the hole 233. Alight receiver 11 is provided at an end portion of a hole 235 formed so as to be opposed to the hole 233 with respect to the container setting part 231. Accordingly, the light outputting end 382 of the light distribution member 380, the condenser lens 234, the container setting part 231, and the light receiver 11 are disposed so as to be linearly arranged. Light outputted from the light outputting end 382 is transmitted, via the condenser lens 234, through the container 15 in the container setting part 231 and the measurement sample in the container 15, to be detected by the light receiver 11. The measurement sample contains a blood specimen and a coagulation time measurement reagent, and is prepared in the container 15. The light receiver 11 outputs, as detection information, a plurality of electric signals (digital data) in accordance with the respective received light intensities.

With reference back to FIG. 2, the sample preparation part 20 includes a dispensing mechanism for dispensing a blood specimen and a coagulation time measurement reagent into a container 15.

As the light applicator 10, the sample preparation part 20, and the detector 230, the light applicator, the sample preparation part, and the detector described in US. Patent No. 10,048,249 can be used, for example. The content of US. Patent No. 10,048,249 is incorporated by reference in the present specification.

### 1-2. Measurement/analysis process

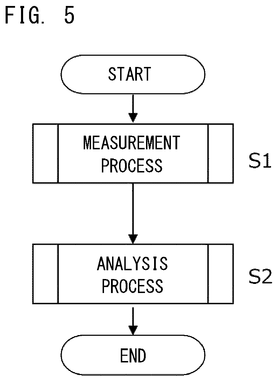

FIG. 5 shows the flow of a measurement/analysis process performed by the controller 201. In step S1, the controller 201 executes a measurement process on the basis of the measurement program 202a. Next, in step S2, the controller 201 executes an analysis process on the basis of the analysis program 202b.

### 1-3. Process of measurement program

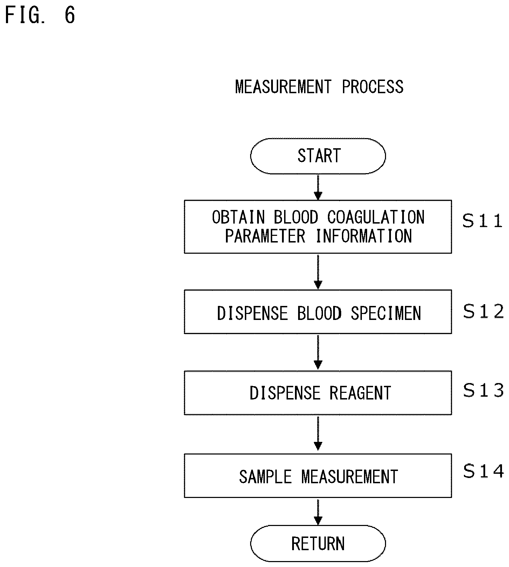

FIG. 6 shows the flow of the measurement process executed by the controller 201 on the basis of the measurement program 202a.

In step S11, the controller 201 obtains, for each blood specimen, information of a blood coagulation parameter (analysis item) ordered for the blood specimen. As the information of the blood coagulation parameter, information inputted by the operator through the display 4 may be received. Alternatively, the information of the blood coagulation parameter may be obtained via a network from, for example, an electronic health record system of a medical facility. The blood specimen and the information of the blood coagulation parameter can be associated with each other by means of an identifier issued at the time of request of a test, for example.

The blood coagulation parameter (analysis item) targeted by the analyzer 1 includes at least one type selected from the group consisting of: activated partial thromboplastin time (hereinafter, this may be abbreviated as "APTT"), prothrombin time (hereinafter, this may be abbreviated as "PT"), thrombo test, fibrinogen, factor II activity, factor V activity, factor VII activity, factor VIII activity, factor IX activity, factor X activity, factor XI activity, factor XII activity, and whole blood coagulation time. Preferably, the blood coagulation parameter can include at least one type selected from the group consisting of APTT and PT.

In step S12, the controller 201 controls the measurement unit 2 so as to dispense a blood specimen into a container 15. At this time, a buffer or the like for diluting the blood specimen may be dispensed into the container 15.

In step S13, the controller 201 controls the sample preparation part 20 of the measurement unit 2 so as to dispense a coagulation time measurement reagent corresponding to each blood specimen to prepare a measurement sample. At this time, when a coagulation activation reagent needs to be added, the coagulation activation reagent is also dispensed. The coagulation time measurement reagent can be selected as appropriate in accordance with each blood coagulation parameter. As the coagulation time measurement reagent, a commercially available reagent can be used.

For example, when APTT is to be measured, an APTT measurement test reagent that can contain: an activator such as silica, ellagic acid, or celite; an animal-derived, plant-derived, or artificially-synthesized phospholipid; and the like, can be used as the coagulation time measurement reagent. Examples thereof can include Thrombocheck APTT series manufactured by Sysmex Corporation, Coagpia (registered trademark) APTT-N, etc., of Sekisui Medical Co., Ltd., and Data-Fi-APTT, etc., of Siemens Healthcare Diagnostics Products GmbH.

The coagulation activation reagent is a reagent that can supply calcium ions. According to the International Committee for Standardization in Hematology, the coagulation activation reagent is a 20 mM calcium chloride solution.

For measurement of PT, a PT measurement test reagent that contains thrombin can be used as the coagulation time measurement reagent. Examples thereof can include Thrombocheck PT series manufactured by Sysmex Corporation, Coagpia (registered trademark) PT series by Sekisui Medical Co., Ltd., and the like. The PT measurement test reagent contains calcium ions necessary for activation of coagulation in general.

In step S14, the controller 201 controls the light applicator 10 so as to start application of light to the container 15 in which the measurement sample has been prepared in step S13. The light receiver 11 continually outputs, as detection information, electric signals (digital data) in accordance with the intensity of light received through the container 15.

### 1-4. Process of analysis program

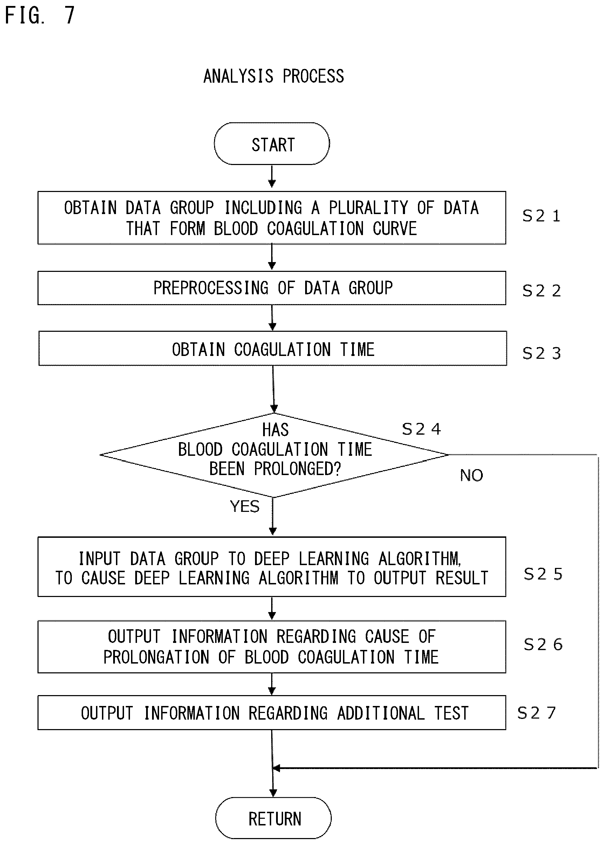

FIG. 7 shows the flow of the analysis process executed by the controller 201 on the basis of the analysis program 202b.

In step S21, the controller 201 obtains a data group including a plurality of data forming a blood coagulation curve. Specifically, the controller 201 arrays, in time series, a plurality of data (digital data) according to the received light intensity outputted from the light receiver 11, and stores the plurality of data into the storage 202.

The controller 201 obtains detection information from the light receiver 11 over time, for example, every 0.1 seconds to 0.5 seconds, and preferably every 0.1 seconds, and stores the detection information into the storage 202. The controller 201 stores, into the storage 202, the plurality of data from the time point when, for example, the blood specimen and the predetermined coagulation time measurement reagent have been added. Generally, after the blood specimen and the coagulation time measurement reagent have been added, a coagulation activation reagent is added. In this case, the controller 201 starts storing the plurality of data from the time point when the coagulation activation reagent has been added. Alternatively, when the coagulation activation reagent has been mixed in the coagulation time measurement reagent, the controller 201 starts storing the plurality of data at the time point when the blood specimen and the coagulation time measurement reagent have been mixed. The controller 201 ends storing the plurality of data at the time point when change in the size of the plurality of data obtained over time is no longer observed. The timings of starting and ending the storing of the plurality of data is not limited thereto. For example, the storing may be started at the time point when light application to the container 15 has been started, and the storing may be ended after a predetermined time (e.g., after 120 seconds or 180 seconds) from the start of the light application.

The data group stored by the controller 201 into the storage 202 may be data obtained by removing a part of the plurality of data outputted from the light receiver 11. As to the removal of a part of data, data of a certain section, such as immediately after the start of the light application or immediately before the end of the light application, may be removed, or data may be removed at a predetermined frequency. Examples of the predetermined frequency can include, for example: when data has been obtained a predetermined number of times, data of the next time is removed; data at even-number times is removed; when data has been obtained a predetermined number of times, data corresponding to a predetermined number of times is removed; and the like.

The controller 201 may arrange the respective data in the data group to be stored in the storage 202, in time series or in an order other than time series, such as according to the intensity of detected light.

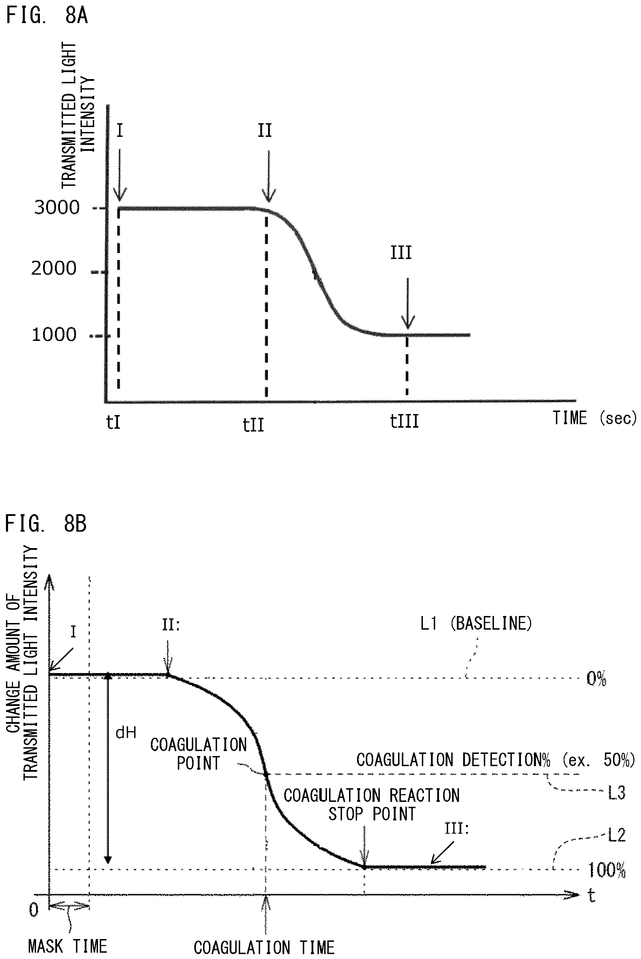

The plurality of data stored in the storage 202 forms a blood coagulation curve. The blood coagulation curve is described in more detail with reference to FIG. 8A. In the present example, the plurality of data indicate the intensity (hereinafter, referred to as "transmitted light intensity") of light, having been applied to a measurement sample, that has been transmitted through the measurement sample. In FIG. 8A, the vertical axis (Y axis) represents transmitted light intensity, and the horizontal axis (X axis) represents measurement time (seconds: sec) at which the transmitted light intensity has been monitored. The blood coagulation curve can be generated by plotting temporal change of the monitored transmitted light intensity in accordance with the two axes of transmitted light intensity and measurement time. The I point in FIG. 8A is the time point at which calcium ions as a coagulation activation reagent and a coagulation time measurement reagent have been added to the test sample, and is also the time point (tl) of measurement start. At the measurement start, fibrinogen in the measurement sample has not changed to fibrin, and deposition of fibrin has not yet occurred in the measurement sample. Thus, the transmitted light intensity indicates a high value. Thereafter, when coagulation reaction is advanced and fibrin begins to be deposited, the deposited fibrin blocks light, whereby the transmitted light intensity begins to decrease. This time point is the II point in FIG. 8A, and is the coagulation start time. The measurement time at which coagulation has started is indicated by (tII). When the reaction is advanced and deposition of fibrin is advanced, the transmitted light intensity decreases accordingly. Most of the fibrinogen in the test sample has changed to fibrin, the reaction is converged, and the change in the transmitted light intensity plateaus. This time point is the III point in FIG. 8A, and is the coagulation end time. The measurement time at which the coagulation has ended is indicated by (till).

In the above, an example in which the light applied to the measurement sample has one wavelength has been described. However, the light applied to the measurement sample may have a plurality of wavelengths. For example, light having a first wavelength due to the first light source 321 may be applied to the measurement sample to obtain a first plurality of data; further, light having a second wavelength due to the second light source 322 may be applied to the same measurement sample to obtain a second plurality of data; further, light having a third wavelength due to the third light source 323 may be applied to the same measurement sample to obtain a third plurality of data; further, light having a fourth wavelength due to the fourth light source 324 may be applied to the same measurement sample to obtain a fourth plurality of data; and further, light having a fifth wavelength due to the fifth light source 325 may be further applied to the same measurement sample to obtain a fifth plurality of data. In this case, the first plurality of data, the second plurality of data, the third plurality of data, the fourth plurality of data, and the fifth plurality of data each form a blood coagulation curve.

In the above embodiment, a plurality of data have been obtained on the basis of the transmitted light intensity. However, the plurality of data can be obtained by any of: an optical measurement method such as of a scattered light type or a transmitted light type; a physical method that uses magnetism and measures viscosity at the time of fibrin deposition; and a dry hematology method. In the case of an optical measurement method, the plurality of data are indicated by signals representing the light amount of transmitted light, scattered light, and the like of the light applied to the measurement sample. In the case of a physical method, the plurality of data are indicated by a signal representing the amplitude of vibration of a steel ball according to the viscosity of the measurement sample.

Next, in step S22 shown in FIG. 7, the controller 201 performs preprocessing on the data group including the plurality of data. The preprocessing includes at least one of smoothing, sharpness, caving, normalization, and differential processing.

Normalization means expressing each data included in the data group, as a relative value so as to be shown between 0% (LI: baseline) and 100% (L2) of the vertical axis of the blood coagulation curve in FIG. 8B. The value obtained through normalization of each data is also referred to as a relative value. The blood coagulation curve obtained by plotting of the relative values is also referred to as a normalized coagulation curve. Normalization can be attained as follows, for example: the change amount (dH) of the transmitted light intensity, i.e., the difference between the transmitted light intensity at the II point as the coagulation reaction start point, and the transmitted light intensity at the III point as the coagulation end time point, is assumed to be 100%; and the change in the transmitted light intensity is expressed as relative values. The plurality of data expressed as relative values form a blood coagulation curve.

When a data group has been obtained by using light having a plurality of wavelengths, the data group expressed as relative values is generated for each wavelength.

In the normalized coagulation curve, the coagulation time can be set as a time point at which the change amount (dH) of the transmitted light intensity is, for example, 30%, 40%, 50%, or 60%. In a preferable embodiment, the coagulation time is the time at which the change amount (dH) of the transmitted light intensity is 50% (L3).

When differential processing is performed on a data group including a plurality of data, the plurality of data included in the data group form a differential curve. The differential curve includes a first-order differential coagulation curve and a second-order differential coagulation curve, for example.

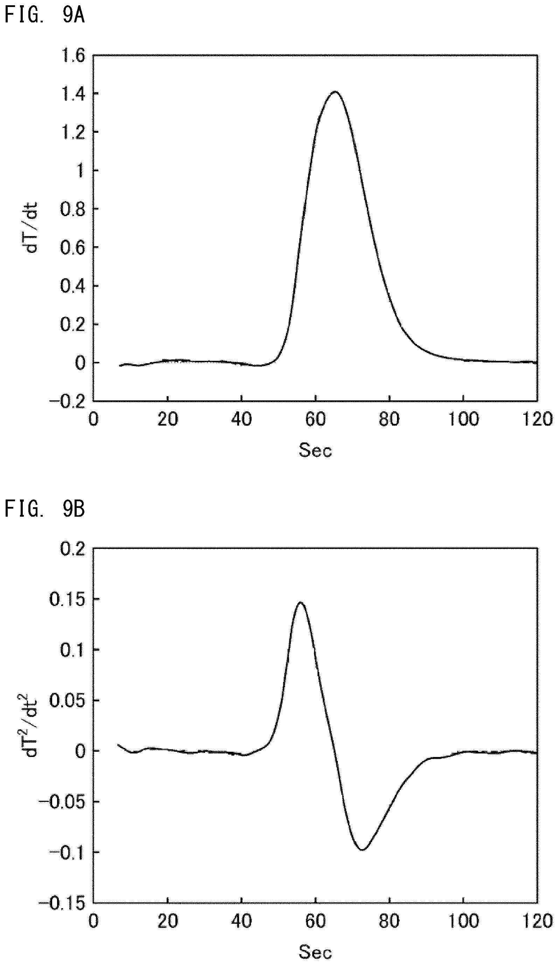

FIG. 9A shows a first-order differential coagulation curve. The first-order differential coagulation curve is obtained by performing first-order differential processing on the blood coagulation curve shown in FIG. 8A or the blood coagulation curve shown in FIG. 8B. The vertical axis (Y axis) data of each set of coordinates forming the first-order differential coagulation curve is also referred to as a first-order differential value.

When a plurality of data groups have been obtained by using light having a plurality of wavelengths, the first-order differential coagulation curve is generated for each wavelength.

FIG. 9B shows a second-order differential coagulation curve. The second-order differential coagulation curve is obtained by performing second-order differential processing on the blood coagulation curve shown in FIG. 8A or the blood coagulation curve shown in FIG. 8B.

When a plurality of data groups have been obtained by using light having a plurality of wavelengths, the second-order differential coagulation curve is generated for each wavelength.

In step S23 shown in FIG. 7, on the basis of the normalized coagulation curve generated in step S22, the controller 201 obtains the coagulation time (the time at which the change amount (dH) of the transmitted light intensity becomes 50% (L3)).

In the present embodiment, step S22 can be omitted. In this case, for example, on the basis of the blood coagulation curve in FIG. 8A, the coagulation time can be calculated as coagulation time (sec)=[(tIII)-(tII)]/2. Here, "-" means subtraction, and "/" means division.

In step S24 shown in FIG. 7, the controller 201 determines whether or not the blood coagulation time has been prolonged. A reference value for the blood coagulation time has been stored in the reference value/threshold DB 202d in advance in accordance with the blood coagulation parameter and the coagulation time measurement reagent. The controller 201 reads out the reference value for the blood coagulation time from the reference value/threshold DB 202d, and compares the reference value with the coagulation time obtained in step S23. When the coagulation time exceeds the reference value ("YES" in step S24), the controller 201 determines that the blood coagulation time has been prolonged, and advances the process to step S25. When the coagulation time does not exceed the reference value ("NO" in step S24), the controller 201 determines that the blood coagulation time has not been prolonged, and returns the process to the measurement/analysis process shown in FIG. 5.

When the coagulation time exceeds the reference value ("YES" in step S24), the controller 201 advances to step S25 shown in FIG. 7, and reads out a deep learning algorithm 60 stored in the algorithm DB 202c in the storage 202. The deep learning algorithm 60 is selected out of a plurality of deep learning algorithms 60 in accordance with the blood coagulation parameter (analysis item) for each blood specimen, and is read out. The controller 201 inputs, to the read-out deep learning algorithm 60, the data group (e.g., normalized coagulation curve, first-order differential coagulation curve, or second-order differential coagulation curve) including the plurality of data obtained in step S22, and obtains a result. Step S22 may be omitted and the data group obtained in step S21 may be inputted to the deep learning algorithm 60 in step S25.

In the following, a method for generating a deep learning algorithm 60 (a method for training a deep learning algorithm 50), and the data group to be inputted to the deep learning algorithm 50 and the deep learning algorithm 60 are described.

### i. Training of deep learning algorithm

The deep learning algorithm is not limited as long as the algorithm has a neural network structure. A convolution neural network, a full connect neural network, and a combination of these can be included.

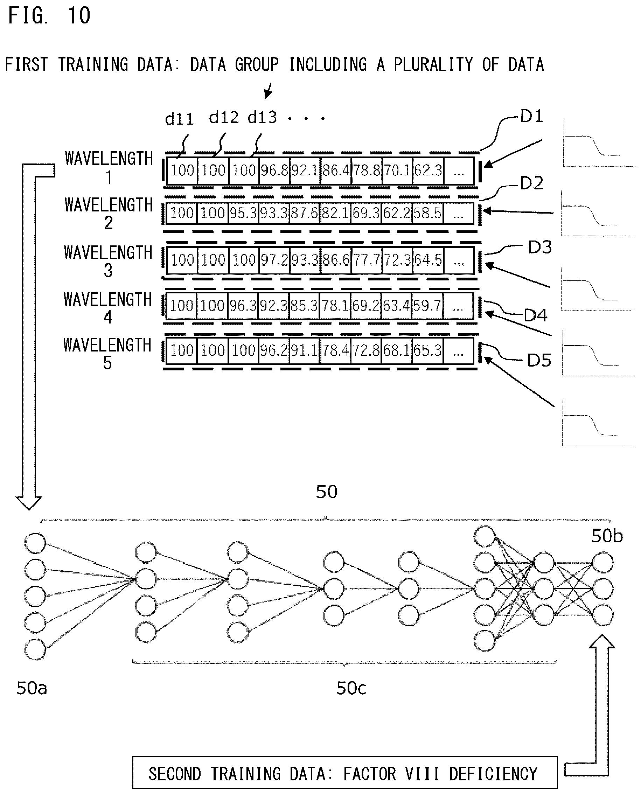

FIG. 10 shows the outline of training of the deep learning algorithm.

As training data for training the deep learning algorithm 50, a data group including a plurality of data obtained from a blood specimen for which the cause of prolongation of blood coagulation time is known is used. The data group is generated according to the method described in steps S21 and S22 shown in FIG. 7 and is used as first training data. As shown in FIG. 10, the data group includes: a first data group D1 corresponding to wavelength 1; a second data group D2 corresponding to wavelength 2; a third data group D3 corresponding to wavelength 3; a fourth data group D4 corresponding to wavelength 4; and a fifth data group D5 corresponding to wavelength 5. The data group D1 includes a plurality of data d11, d12, d13,

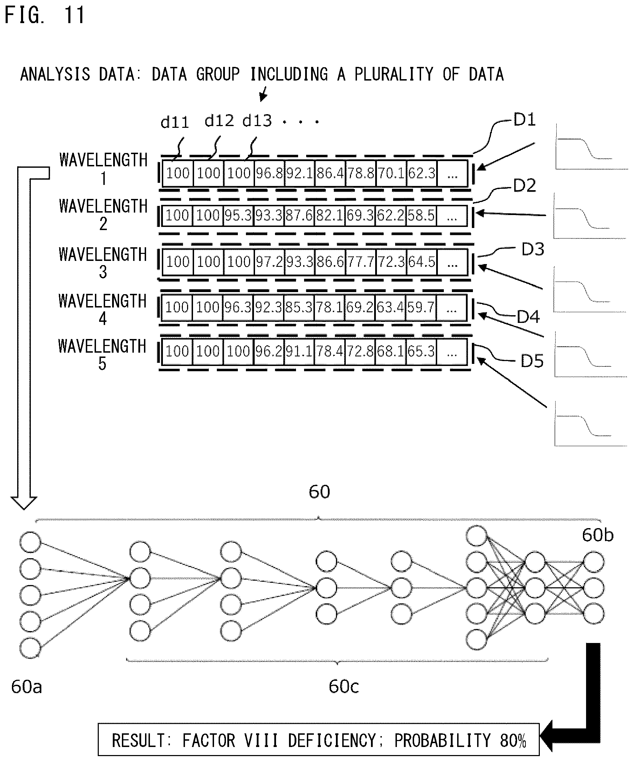

Similarly, the data group D2 to data group D5 each include a plurality of data. The first training data is inputted to an input layer 50a of the neural network 50 shown in FIG. 10, and a label (in the example in FIG. 10, "factor VIII deficiency") indicating the cause of the prolongation of the blood coagulation time and corresponding to the inputted first training data is inputted, as second training data, to an output layer 50b. On the basis of these inputs, for each blood specimen, the first training data and the second training data are associated with each other, and a weight for each layer in a middle layer 50c of the neural network 50 is calculated, whereby a trained deep learning algorithm 60 shown in FIG. 11 is generated.

Training of the deep learning algorithm 50 may be performed for each blood coagulation parameter, or a plurality of blood coagulation parameters, such as a first blood coagulation parameter and a second blood coagulation parameter, may be combined. For example, the deep learning algorithm 50 may be trained by using only a data group including a plurality of data, obtained through APTT measurement, which have been obtained from a blood specimen for which the cause of prolongation of blood coagulation time is known. In this case, the trained deep learning algorithm 60 becomes a deep learning algorithm 60 for performing analysis based on the data group including the plurality of data derived from APTT. The deep learning algorithm 50 may be trained by using only a data group including a plurality of data, obtained through PT measurement, which have been obtained from a blood specimen for which the cause of prolongation of blood coagulation time is known. In this case, the trained deep learning algorithm 60 becomes a deep learning algorithm 60 that performs analysis on the basis of the data group including the plurality of data derived from PT. Further, the deep learning algorithm 50 may be trained by using a data group including a plurality of data obtained through measurement of APTT as the first blood coagulation parameter, and a data group including a plurality of data obtained through measurement of PT as the second blood coagulation parameter. In this case, the trained deep learning algorithm 60 becomes a deep learning algorithm 60 that performs analysis on the basis of the data group including the plurality of data obtained through APTT measurement and the data group including the plurality of data derived from PT.

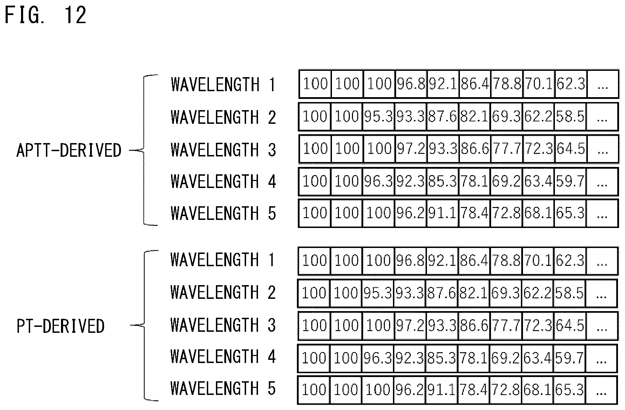

When a plurality of blood coagulation parameters for training are combined to produce the first training data, as shown in FIG. 12, for example, a data group including a plurality of data derived from the first blood coagulation parameter and a data group including a plurality of data derived from the second blood coagulation parameter may be arrayed in this order, and then, inputted as the first training data.

As shown in FIG. 12, a data group composed of a data group including a plurality of data corresponding to the first wavelength derived from the first blood coagulation parameter (e.g., APTT), followed by data groups including a plurality of data corresponding to the second wavelength, the third wavelength, the fourth wavelength, and the fifth wavelength; and a data group composed of a data group including a plurality of data corresponding to the first wavelength derived from the second blood coagulation parameter (e.g., PT), followed by data groups corresponding to the second wavelength, the third wavelength, the fourth wavelength, and the fifth wavelength, are arrayed in this order, and then, this resultant data can be inputted as the first training data.

The first training data and the second training data are generated from each of a plurality of blood specimens for each of which the cause of prolongation of blood coagulation time is known, and are used for training the deep learning algorithm 50.

### ii. Analysis by trained deep learning algorithm

A data group including a plurality of data, which form a blood coagulation curve and which have been obtained from a blood specimen to be analyzed, is inputted as analysis data to an input layer 60a of the trained deep learning algorithm 60 in FIG. 11. The blood specimen to be analyzed may be a blood specimen, collected from a patient, for which the presence or absence of prolongation of blood coagulation time is not known; or may be a blood specimen, collected from a patient, for which the presence of prolongation of blood coagulation time is known.

Preferably, the analysis data to be inputted is a data group including a plurality of data regarding the same blood coagulation parameter and having the same configuration as those of the first training data used when training the deep learning algorithm 60. For example, as shown in FIG. 11, the data group includes: a first data group D1 corresponding to wavelength 1; a second data group D2 corresponding to wavelength 2; a third data group D3 corresponding to wavelength 3; a fourth data group D4 corresponding to wavelength 4; and a fifth data group D5 corresponding to wavelength 5. The data group D1 includes a plurality of data d11, d12, d13,

Similarly, the data group D2 to data group D5 each include a plurality of data. That the first training data and the analysis data have the same configuration means that the analysis data is a data group including a plurality of data obtained using light having the same wavelengths as those of the first training data. When partial data has been removed from the data group including the plurality of data used in the first training data, a similar process is preferably performed also on the analysis data. Further, when preprocessing has been performed on the first training data, similar preprocessing is preferably performed also on the analysis data.

When there are a plurality of deep learning algorithms 60 corresponding to blood coagulation parameters (analysis items), a deep learning algorithm 60 to which inputting is performed may be selected from the plurality of deep learning algorithms in accordance with the kind of the blood coagulation parameter.

When the cause of prolongation is to be predicted on the basis of a plurality of blood coagulation parameters, as shown in FIG. 12, for example, a data group composed of a data group including a plurality of data corresponding to the first wavelength derived from the first blood coagulation parameter (e.g., APTT), followed by data groups including a plurality of data corresponding to the second wavelength, the third wavelength, the fourth wavelength, and the fifth wavelength; and a data group composed of a data group including a plurality of data corresponding to the first wavelength derived from the second blood coagulation parameter (e.g., PT), followed by data groups corresponding to the second wavelength, the third wavelength, the fourth wavelength, and the fifth wavelength, are arrayed in this order, and then, this resultant data can be inputted to the deep learning algorithm 60.

The deep learning algorithm 60 outputs a result (in FIG. 11, "factor VIII deficiency; 80%") from an output layer 60b. The result can include: a label (in the example in FIG. 11, "factor VIII deficiency") indicating the cause of the prolongation of the blood coagulation time; and a probability of belonging to the label (in FIG. 11 "80%").

Here, the label indicating the cause of the prolongation of the blood coagulation time and inputted as the second training data, and the label indicating the cause of the prolongation of the blood coagulation time and outputted as a result may be character information or a label value.

The cause of the prolongation of the blood coagulation time outputted from the output layer 60b of the deep learning algorithm 60 can include at least one selected from the group consisting of: liver disease; disseminated intravascular coagulation (hereinafter, this may be abbreviated as "DIC"); vitamin K (hereinafter, this may be abbreviated as "VK") deficiency; hemorrhaging; decrease, deficiency, or dysfunction of a coagulation factor; presence of a coagulation factor inhibitor; presence of lupus anticoagulant (hereinafter, this may be abbreviated as "LA"); use of an anticoagulant drug; presence of an abnormal protein such as in macroglobulinemia; and a cause derived from a blood collection technique.

Decrease, deficiency, or dysfunction of a coagulation factor can include decrease, deficiency, or dysfunction of at least one selected from the group consisting of fibrinogen (hereinafter, this may be abbreviated as "Fbg"), factor II (hereinafter, this may be abbreviated as "FII"), factor V (hereinafter, this may be abbreviated as "FV"), factor VII (hereinafter, this may be abbreviated as "FVII"), factor VIII (hereinafter, this may be abbreviated as "FVIII"), von Willebrand factor (hereinafter, this may be abbreviated as "VWF"), factor IX (hereinafter, this may be abbreviated as "FIX"), factor X (hereinafter, this may be abbreviated as "FX"), factor XI (hereinafter, this may be abbreviated as, "FXI"), factor XII (hereinafter, this may be abbreviated as "FXII"), HMWK (High Molecular Weight Kininogen), and prekallikrein.

The coagulation factor inhibitor can include at least one selected from the group consisting of a factor V inhibitor, a factor VIII inhibitor, a von Willebrand factor inhibitor, and a factor IX inhibitor.

The anticoagulant drug is also referred to as an antithrombotic drug. The anticoagulant drug can include: a coumarin-based drug such as warfarin potassium; heparin; a synthetic Xa inhibitor such as fondaparinux sodium; an oral direct Xa inhibitor such as edoxaban tosilate hydrate and apixaban; an oral thrombin direct inhibitor such as dabigatran etexilate methanesulfonate; an antithrombic agent such as argatroban hydrate; and the like.

When the predetermined blood coagulation parameter is thrombo test or whole blood coagulation time, the anticoagulant drug can include an antiplatelet drug. The antiplatelet drug can include ticlopidine hydrochloride, clopidogrel sulfate, prasugrel hydrochloride, ticagrelor, a clopidogrel sulfate/aspirin combination drug, cilostazol, ethyl icosapentate, beraprost sodium, sarpogrelate hydrochloride, an aspirin/dialuminate combination drug, aspirin, and the like.

Examples of the cause derived from a blood collection technique can include: a case of contamination of heparin during blood collection such as line blood collection or post-dialysis blood collection; a case of a small blood collection amount relative to the proportion of an anticoagulant agent filled in advance in a blood collection tube; a case where the blood vessel is thin and blood collection is difficult, resulting in contamination of tissue fluid during blood collection; a case where time has elapsed from blood collection; and the like.

The cause of the prolongation of the blood coagulation time outputted from the output layer 60b of the deep learning algorithm 60 is, preferably, at least one selected from the group consisting of a cause related to prolongation of activated partial thromboplastin time, and a cause related to prolongation of prothrombin time.

Next, the controller 201 advances to step S26 shown in FIG. 7, and outputs, to the display 4 or the network 99, information regarding the cause of the prolongation of the blood coagulation time, on the basis of the result outputted from the deep learning algorithm 60. The output in step S26 may be performed after the controller 201 has received an output start request inputted by the operator through the display 4.

The information regarding the cause of the prolongation of the blood coagulation time can include at least a label indicating a cause candidate for the prolongation of the blood coagulation time. Preferably, said information can include a probability that the cause candidate indicated by the label is a cause of the prolongation of the blood coagulation time. The label indicating a cause candidate for the prolongation of the blood coagulation time may be character information or a label value. The character information can include a notation in the form of an abbreviation or the like such as FIX or FVIII. The label value can include a notation in the form of a symbol, such as 1, 2, or the like, associated in advance with a predetermined prolongation cause.

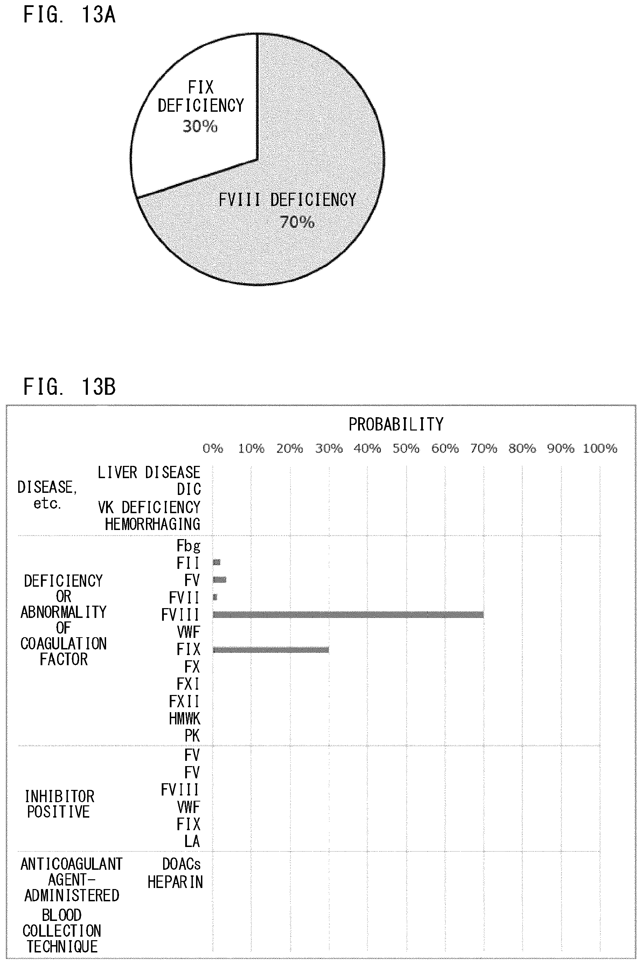

The probability that the cause candidate is a cause of the prolongation of the blood coagulation time may be expressed in a softmax form or a binary form. The softmax form is a form that indicates the probability that, among a plurality of cause candidates for prolongation of blood coagulation time, a predetermined cause candidate is a cause of the prolongation of the blood coagulation time. For example, when the prolongation cause outputted from the deep learning algorithm 60 includes only factor VIII deficiency and factor IX deficiency as the causes whose probability is greater than 0%, the probabilities of the respective prolongation causes are indicated such that the total of these probabilities becomes 100%. The binary form is a form in which the probability that each of a plurality of prolongation cause candidates is a cause of the prolongation of the blood coagulation time, and the total of the probabilities of the prolongation cause candidates does not necessarily become 100%. The probability that a cause candidate is a cause of the prolongation of the blood coagulation time may be outputted in a form of a graph as shown, for example, in FIG. 13A or FIG. 13B. FIG. 13A is an example in which the probabilities indicated by a softmax form are outputted as a pie graph. FIG. 13B is an example in which the probabilities indicated by a binary form are outputted as a bar graph.

When the information regarding the cause of the prolongation of the blood coagulation time is to be outputted, a label indicating a cause candidate, for the prolongation of the blood coagulation time, for which the probability is the highest may be outputted, for example. In this case, for example, in the examples of FIG. 13A and FIG. 13B, a label that indicates FVIII deficiency having the highest probability is outputted.

When the information regarding the cause of the prolongation of the blood coagulation time is to be outputted, a label indicating a cause candidate, for the prolongation of the blood coagulation time, for which the probability is not less than a predetermined threshold may be outputted, for example. The threshold can be 10%, 20%, 30%, 40%, 50%, 60%, 70%, 80%, or 90%, for example. In the examples of FIG. 13A and FIG. 13B, when, for example, 20% is set as the threshold, FIX deficiency and FVIII deficiency are outputted as the prolongation cause, and when, for example, 60% is set as the threshold, FVIII deficiency is outputted as the prolongation cause.

In a case where the information regarding the cause of the prolongation of the blood coagulation time is to be outputted, when a threshold is to be used, the controller 201 can retrieve the threshold from the reference value/threshold DB 202d stored in the storage 202. The threshold may be inputted by the operator through the display 4, and the controller 201 may receive the threshold.

In step S27 shown in FIG. 7, the controller 201 reads out information regarding an additional test from the additional test DB 202e stored in the storage 202, and outputs the information from the display 4. The information regarding the additional test is outputted on the basis of the result outputted from the deep learning algorithm 60 in step S25. The additional test is at least one selected from the group consisting of a test regarding a coagulation factor, a test regarding a coagulation factor inhibitor, and a re-test regarding a measurement item of measurement performed on the measurement sample. The additional test is a test performed for confirming or re-testing the result outputted from the deep learning algorithm 60.

For example, among the causes of the prolongation of the blood coagulation time outputted by the deep learning algorithm 60, when the probability that the cause is liver disease is the highest, the additional test can be a biochemical test (AST, ALT, γ-GTP, LDH, ALP, etc.) for evaluating the liver function.

Among the causes of the prolongation of the blood coagulation time outputted by the deep learning algorithm 60, when the probability that the cause is disseminated intravascular coagulation is the highest, the additional test can be platelet number measurement, blood fibrinogen concentration measurement, FDP/D-dimer measurement, blood thrombin-antithrombin complex concentration measurement, measurement of a fibrinolytic system marker, such as plasmin-α2 plasmin inhibitor complex, or the like.

Among the causes of the prolongation of the blood coagulation time outputted by the deep learning algorithm 60, when the probability that the cause is vitamin K deficiency is the highest, the additional test can be blood vitamin K concentration measurement.

Among the causes of the prolongation of the blood coagulation time outputted by the deep learning algorithm 60, when the probability that the cause is hemorrhaging is the highest, the additional test can be red blood cell number measurement, hemoglobin concentration measurement, hematocrit value measurement, platelet number measurement, or the like.

Among the causes of the prolongation of the blood coagulation time outputted by the deep learning algorithm 60, when the probability that the cause is decrease, deficiency, or dysfunction of a certain coagulation factor is the highest, the additional test can be: measurement (including a blood coagulation method, a synthetic substrate method, etc.) of the activity of the certain coagulation factor; measurement of the concentration of protein of the certain coagulation factor by an enzyme immunoassay, etc.; or the like.

Among the causes of the prolongation of the blood coagulation time outputted by the deep learning algorithm 60, when the probability that the cause is the presence of a certain coagulation factor inhibitor is the highest, the additional test can be: detection of the certain coagulation factor inhibitor, for example, detection of an anti-coagulation factor antibody by an enzyme immunoassay, etc.; a mixing test; a cross-mixing test; or the like.

Among the causes of the prolongation of the blood coagulation time outputted by the deep learning algorithm 60, when the probability that the cause is the presence of lupus anticoagulant is the highest, the additional test can be: detection of an anti-phospholipid antibody by an enzyme immunoassay, etc.; a cross-mixing test; or the like.

When the deep learning algorithm 60 has outputted that the cause of the prolongation of the blood coagulation time is the presence of an abnormal protein, the additional test can be serum protein analysis.

Among the causes of the prolongation of the blood coagulation time outputted by the deep learning algorithm 60, when the probability that the cause is an anticoagulant drug is the highest, the additional test can be confirmation of the medication history of the subject from whom the blood sample has been collected.

Among the causes of the prolongation of the blood coagulation time outputted by the deep learning algorithm 60, when the probability that the cause is a cause derived from the blood collection technique is the highest, the additional test can be a re-test including recollection of a blood sample.

The information regarding the additional test may be a label value or character information indicating the name of the additional test.

In the output of information regarding the additional test, in addition to the additional test that corresponds to the prolongation cause having the highest probability, an additional test that corresponds to the prolongation cause having the second highest probability or lower may be outputted. In this case, a priority ranking of each additional test may be outputted.

It should be noted that step S27 can be omitted.

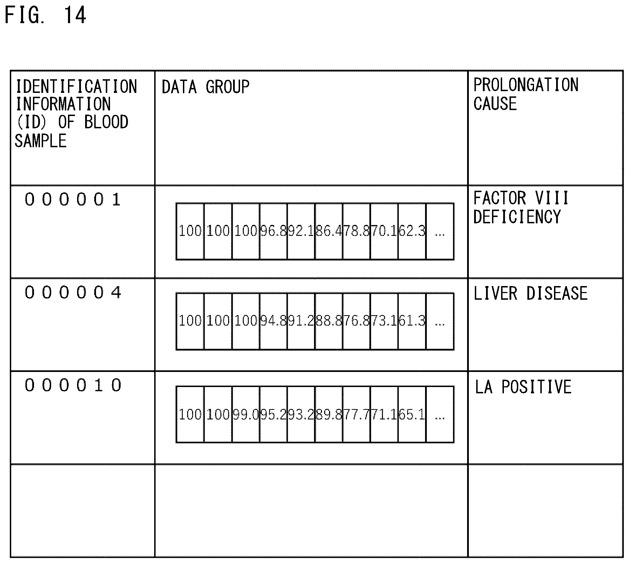

Further, when a more specific cause of the prolongation of the blood coagulation time has been identified by the additional test, the operator may input, through the display 4, the identified prolongation cause in association with identification information of the blood sample for which the additional test has been performed. The inputted prolongation cause may be stored into the additional test DB 202e of the storage 202, so as to be associated with the identification information of the blood sample and the data group including the plurality of data with respect to the blood sample. FIG. 14 shows an example of the identification information of the blood sample, the data group, and the prolongation cause stored in the additional test DB 202e. The identification information of the blood sample, the data group, and the prolongation cause stored in the additional test DB 202e may be transmitted via the communication I/F 208, to another computer in the network 99, for example, a training computer for further training the deep learning algorithm 60. Accordingly, the training computer can further train the deep learning algorithm 60 on the basis of the received information, and can further improve the analysis ability of the deep learning algorithm 60.

### 2. Training apparatus

### 2-1. Configuration of training apparatus

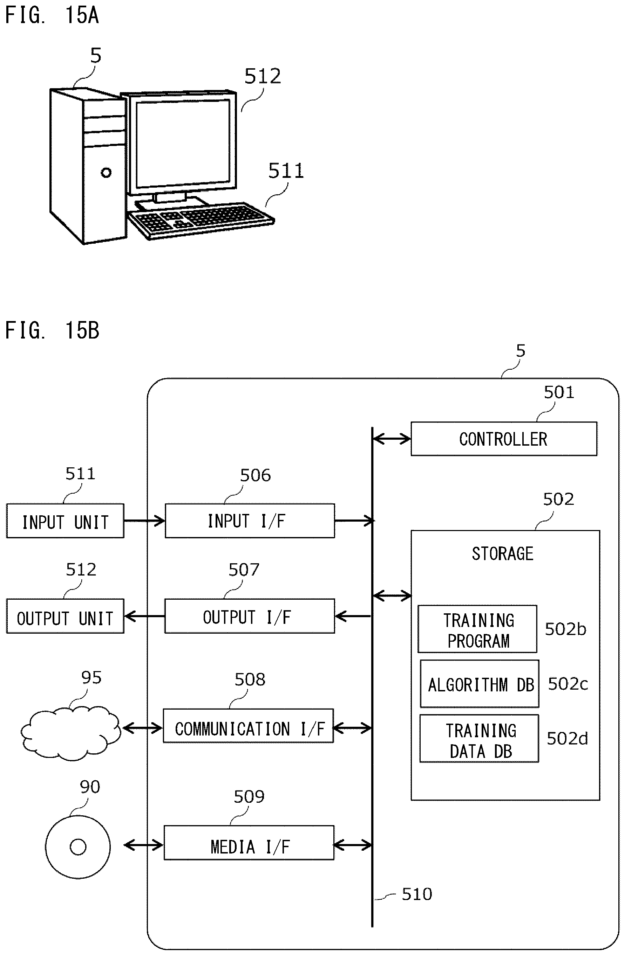

An embodiment disclosed in the present specification relates to a training apparatus 5 (hereinafter, simply referred to as a "training apparatus 5") of the deep learning algorithm 50. FIG. 15A shows an example of the appearance of the training apparatus 5. The training apparatus 5 is communicably connected to an input unit 511 and an output unit 512. The training apparatus 5 is a so-called general-purpose computer.

FIG. 15B shows an example of a hardware configuration of the training apparatus 5. The training apparatus 5 includes a controller 501, a storage 502, an input interface (I/F) 506, an output interface (I/F) 507, a communication interface (I/F) 508, a media interface (I/F) 509, and a bus 510.

The controller 501 includes an arithmetic processing device such as a CPU.

The storage 502 stores: a training program 502b described later; an algorithm database (DB) 502c storing one or a plurality of deep learning algorithms 50 and/or deep learning algorithms 60; and a training data database (DB) 502d storing the first training data and the second training data so as to be associated with each other.

The input interface 506 receives input information inputted by the operator through the input unit 511 implemented as a touch panel, a keyboard, or the like, and transmits the received input information to the controller 501 or the storage 502.

The output interface 507 transmits output information outputted by the controller 501, to the output unit 512 such as a display.

The communication interface 508 communicably connects the training apparatus 5 to a network 95. The connection may be wired connection or wireless connection.

The media interface 509 performs transmission of information with respect to a nonvolatile storage medium such as a CD-ROM, a DVD-ROM, an external hard disk, or the like.

Signal transmission in the training apparatus 5 is performed via the bus 510.

### 2-2. Process of training program

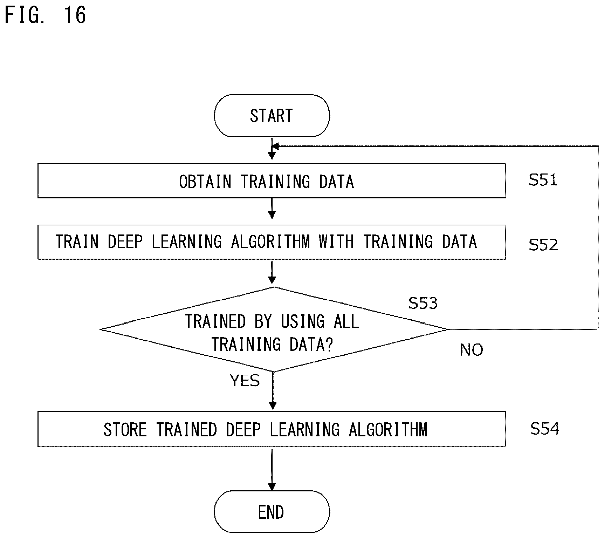

FIG. 16 shows an example of a training process executed by the training program 502b.

In step S51, the controller 501 receives a process start request inputted by the operator through the input unit 511, and retrieves training data from the training data DB 502d.

Subsequently, in step S52, the controller 501 retrieves a deep learning algorithm 50 or a deep learning algorithm 60 from the algorithm DB 502c in the storage 502, inputs the training data to the retrieved deep learning algorithm, and performs training. Details of the training have been described in "i. Training of deep learning algorithm". Inputting training data to the deep learning algorithm 60 to train the deep learning algorithm 60 is also referred to as retraining of the deep learning algorithm 60.

Next, in step S53, the controller 501 determines whether or not the deep learning algorithm 50 or the deep learning algorithm 60 has been trained by using all of the training data that should be used. When training has been performed by using all of the training data ("YES" in step S53), the controller 501 advances to step S54, and stores the trained deep learning algorithm 60 into the algorithm DB 502c stored in the storage 202.

In step S53, when training has not been performed by using all of the training data ("NO" in step S53), the controller 501 returns to step S51 and continues the training process.

### 3. Storage medium having stored therein analysis program or training program

The analysis program 202b and/or the training program 502b can be provided as a program product such as a storage medium. The computer program is stored in a storage medium such as a hard disk, a semiconductor memory device such as a flash memory, or an optical disk. The storage form of the program in the storage medium is not limited as long as the controller can read the program.

It should be noted that, in the present specification, the anticoagulant agent and the anticoagulant drug are used so as to be distinguished from each other. The anticoagulant agent is an agent that is filled in a blood collection tube or a syringe in order to prevent deposition of fibrin during blood collection. For example, usually, when blood is collected to be used in a coagulation test, the collection is preferably performed by using, as the anticoagulant agent, a citrate, e.g., a sodium citrate solution. The blood specimen is prepared by, for example, using a 3.1% to 3.3% (weight/volume) trisodium citrate solution as an anticoagulant agent and mixing this anticoagulant agent and whole blood at a volume ratio of about 1:8.5 to 1:9.5. Alternatively, the blood specimen may be plasma separated from a mixture of the anticoagulant agent and whole blood. 4. Modification

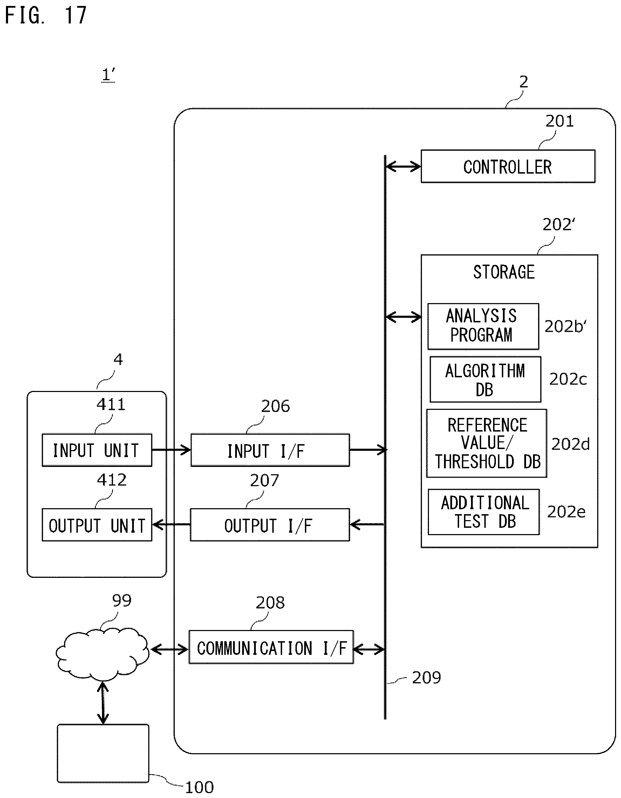

FIG. 17 shows an analyzer 1' as a modification of the analyzer 1. The analyzer 1' is a computer of a cloud service provider, for example, and is connected to a known blood analyzer 100 via a network 99. The analyzer 1' includes a controller 201, a storage 202', an input interface (I/F) 206, an output interface (I/F) 207, a communication interface (I/F) 208, and a bus 209. As the controller 201, the input interface (I/F) 206, the output interface (I/F) 207, the communication interface (I/F) 208, and the bus 209, those that are the same as those of the analyzer 1 can be used. As the storage 202', a storage obtained by removing the measurement program 202a from the storage 202 of the analyzer 1 and changing the analysis program 202b to the analysis program 202b' can be used.

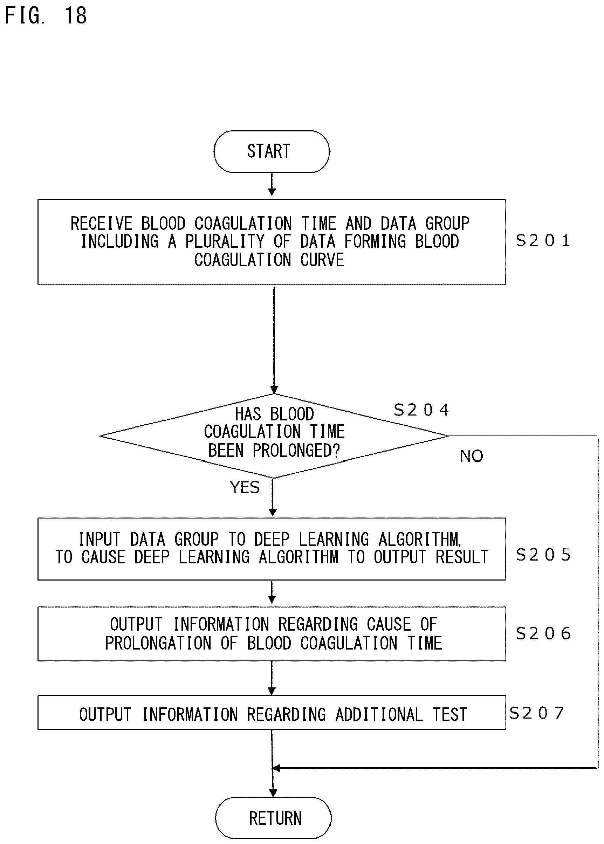

FIG. 18 shows the flow of a process executed on the basis of the analysis program 202b' by the controller 201 of the analyzer 1'. In step S201, the controller 201 receives, from the analyzer 100, a data group including a plurality of data forming a blood coagulation curve, and a blood coagulation time. The data group that is received is a data group similar to the data group obtained in step S22 in FIG. 7. The coagulation time that is received is a coagulation time similar to the coagulation time obtained in step S23 in FIG. 7. Thereafter, the controller 201 executes steps S204 to S207. Steps S204 to S207 are processes that are respectively similar to steps S24 to S27 in FIG. 7, and thus description thereof is omitted.

As the known blood analyzer 100, a fully automated blood coagulation measurement apparatus CN-6000, CN-3000, or the like manufactured by Sysmex Corporation can be used, for example.

### 5. Examination of effect

### 5-1. Comparison with conventional method

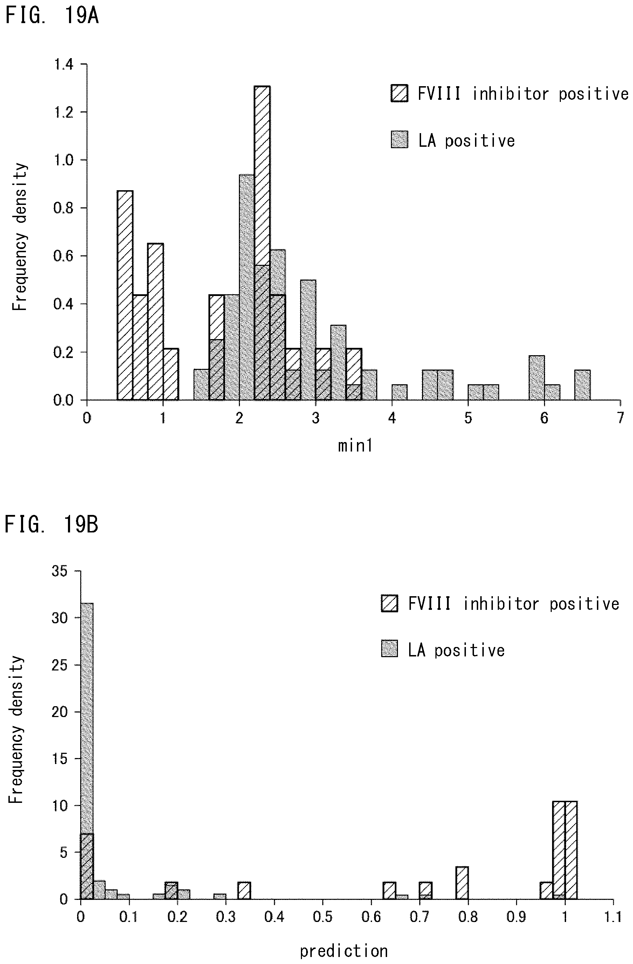

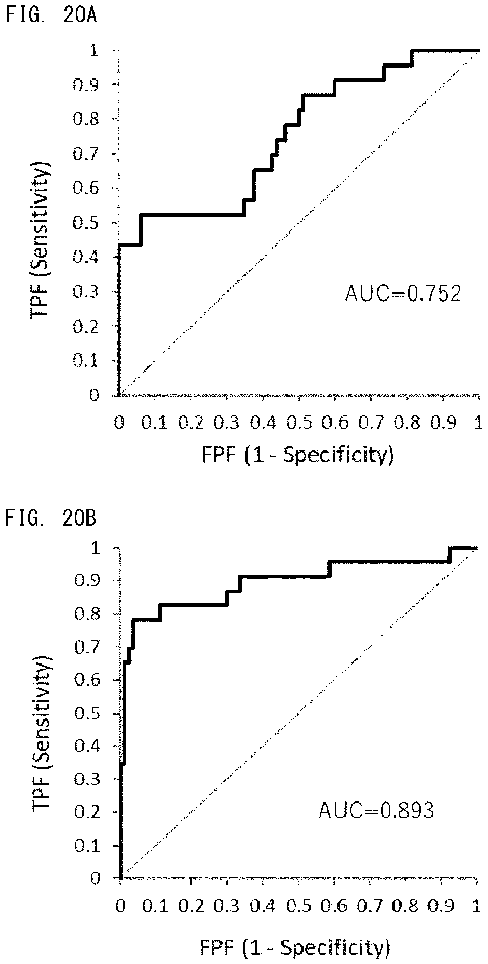

APTT measurement was performed by using specimens (plasma) for which the APTT prolongation cause is the presence of lupus anticoagulant (LA), and specimens for which the APTT prolongation cause is the presence of a factor VIII inhibitor (FVIII inhibitor). Using the measurement result, the prediction accuracy of a conventional method (first-order differential method) and the prediction accuracy of the present analysis method executed by the analyzer 1 were compared with each other. FIGS. 19A, 19B and FIGS. 20A, 20B show the results. FIG. 19A shows a histogram in which the horizontal axis represents the peak value (mini) in the first-order differential curve of each specimen. FIG. 19B is a histogram in which the horizontal axis represents the probability that the APTT prolongation cause outputted by the present analysis method is the presence of a factor VIII inhibitor. In the conventional method, at min1=1.5 to min1=3.5, LA positive specimens and factor VIII inhibitor-containing specimens overlap each other, and are not separated from each other. Meanwhile, in the present analysis method, LA positive specimens and factor VIII inhibitor-containing specimens were able to be separated from each other. This shows that the present analysis method can predict the cause of prolongation of the blood coagulation time, with respect to specimens (specimen exhibiting min1=1.5 to mini =3.5) for which the conventional method failed in the prediction.

FIG. 20A shows an ROC curve according to the conventional method with respect to the specimens shown in FIG. 19. FIG. 20B shows an ROC curve with which the prediction accuracy of the present analysis method has been evaluated. In the conventional method, AUC=0.752, and in the present analysis method, AUC=0.893. According to these results, it has been shown that the present analysis method has higher sensitivity and higher specificity than the conventional method.

### 5-2. Evaluation of PT deep learning algorithm

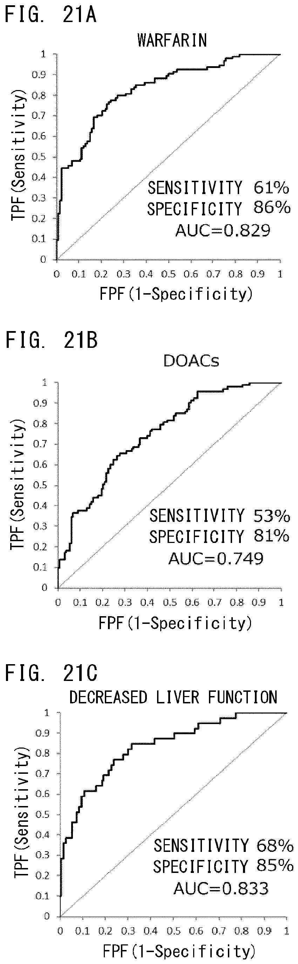

FIGS. 21A, 21B, 21C show ROC curves obtained when: PT measurement was performed on 94 warfarin-administered blood specimens, 93 DOACs-administered blood specimens, and 39 decreased liver function blood specimens; and the present analysis method was executed by the analyzer 1. With respect to the warfarin-administered blood specimens (FIG. 21A) and the decreased liver function blood specimens (FIG. 21C), prediction was able to be performed with an accuracy having an AUC exceeding 0.80. With respect to the DOACs-administered blood specimens (FIG. 21B), prediction was able to be performed with an accuracy having an AUC exceeding 0.74 although lower than those of the warfarin-administered blood specimens and the decreased liver function blood specimens.

### 5-3. Evaluation of APTT deep learning algorithm

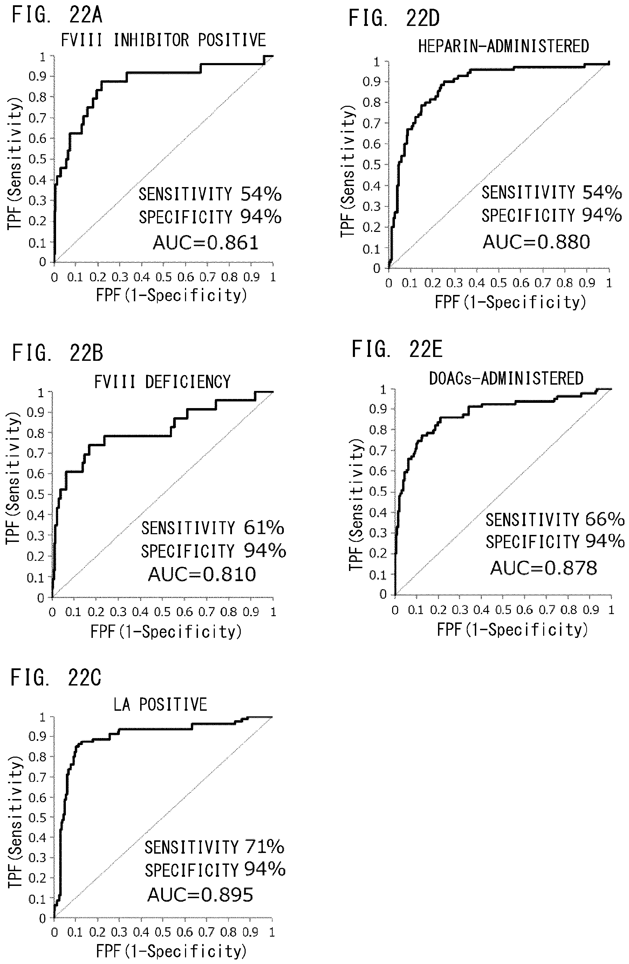

FIGS. 22A, 22B, 22C, 22D, 22E show ROC curves obtained when: APTT measurement was performed on 24 factor VIII (FVIII) inhibitor positive blood specimens, 23 factor VIII (FVIII) deficient blood specimens, 80 LA positive blood specimens, 70 heparin-administered blood specimens, and 79 DOACs-administered blood specimens; and the present analysis method was executed by the analyzer 1. In each of the above, prediction was able to be performed with an accuracy having an AUC exceeding 0.80.

### 5-4. Evaluation of PT and APTT deep learning algorithm

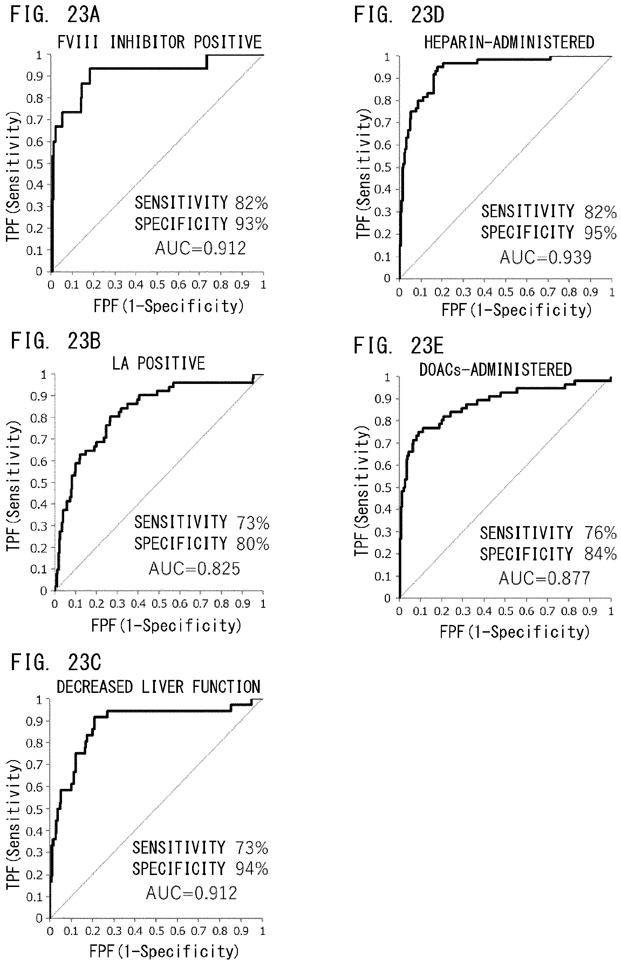

FIGS. 23A, 23B, 23C, 23D, 23E show ROC curves obtained when: PT measurement and APTT measurement were performed on 15 factor VIII (FVIII) inhibitor positive blood specimens, 51 LA positive blood specimens, 36 decreased liver function blood specimens, 60 heparin-administered blood specimens, and 56 DOACs-administered blood specimens; and the present analysis method was executed by the analyzer 1. In each of the above, prediction was able to be performed with an accuracy having an AUC exceeding 0.82.

## Claims

1. An analysis method for a blood specimen, comprising:
obtaining a data group including a plurality of data forming a blood coagulation curve or a differential curve thereof;
inputting the data group into a deep learning algorithm; and
outputting, on the basis of a result obtained from the deep learning algorithm, information regarding a cause of prolongation of blood coagulation time of the blood specimen.

2. The analysis method of claim 1, wherein
the plurality of the data included in the data group form the blood coagulation curve.

3. The analysis method of claim 1, wherein
the plurality of the data included in the data group form a first-order differential curve or a second-order differential curve of the blood coagulation curve.

4. The analysis method of any one of claims 1 to 3, wherein
the plurality of the data included in the data group are obtained at a predetermined interval between start and end of coagulation reaction.

5. The analysis method of any one of claims 1 to 4, wherein
the plurality of the data included in the data group are obtained through optical measurement.

6. The analysis method of claim 5, wherein
the data group includes a plurality of the data obtained by applying light having a first wavelength to a measurement sample that contains the blood specimen and a coagulation time measurement reagent; and a plurality of the data obtained by applying light having a second wavelength different from the first wavelength to the measurement sample.

7. The analysis method of any one of claims 1 to 6, wherein
the data group includes a first data group with respect to a first blood coagulation parameter, and a second data group with respect to a second blood coagulation parameter.

8. The analysis method of any one of claims 1 to 7, wherein
the cause of the prolongation of the blood coagulation time comprises at least one selected from the group consisting of a cause related to prolongation of activated partial thromboplastin time, and a cause related to prolongation of prothrombin time.

9. The analysis method of claim 8, wherein
the cause of the prolongation of the blood coagulation time comprises at least one selected from the group consisting of liver disease; disseminated intravascular coagulation; vitamin K deficiency; hemorrhaging; decrease, deficiency, or dysfunction of a coagulation factor; presence of a coagulation factor inhibitor; presence of lupus anticoagulant; use of an anticoagulant drug; presence of an abnormal protein; and a cause derived from a blood collection technique.

10. The analysis method of claim 9, wherein
the decrease, deficiency, or dysfunction of the coagulation factor comprises decrease, deficiency, or dysfunction of at least one selected from the group consisting of fibrinogen, factor II, factor V, factor VII, factor VIII, von Willebrand factor, factor IX, factor X, factor XI, factor XII, HMWK (High Molecular Weight Kininogen), and prekallikrein.

11. The analysis method of claim 9, wherein
the coagulation factor inhibitor comprises at least one selected from the group consisting of a factor V inhibitor, a factor VIII inhibitor, a von Willebrand factor inhibitor, and a factor IX inhibitor.

12. The analysis method of claim 9, wherein
the cause derived from the blood collection technique includes contamination of heparin.

13. The analysis method of any one of claims 1 to 12, wherein
the information regarding the cause of the prolongation of the blood coagulation time includes: a label indicating a cause candidate for the prolongation of the blood coagulation time; and a probability that the cause candidate indicated by the label is a cause of the prolongation of the blood coagulation time.

14. The analysis method of any one of claims 1 to 12, wherein
the information regarding the cause of the prolongation of the blood coagulation time includes: a label indicating a cause candidate for the prolongation of the blood coagulation time; and a probability that, among a plurality of cause candidates for prolongation of blood coagulation time, the cause candidate indicated by the label is a cause of the prolongation of the blood coagulation time.

15. The analysis method of claim 13 or 14, wherein
the outputting of the information regarding the cause of the prolongation of the blood coagulation time comprises displaying the probability in a form of a graph.

16. The analysis method of any one of claims 13 to 15, wherein
the outputting of the information regarding the cause of the prolongation of the blood coagulation time comprises outputting a label indicating a cause candidate, for the prolongation of the blood coagulation time, for which the probability is highest.

17. The analysis method of any one of claims 13 to 15, wherein
the outputting of the information regarding the cause of the prolongation of the blood coagulation time comprises outputting a label indicating a cause candidate, for the prolongation of the blood coagulation time, for which the probability is not less than a predetermined threshold.

18. The analysis method of claim 17, further comprising
receiving setting of the predetermined threshold.

19. The analysis method of any one of claims 1 to 18, further comprising
outputting information regarding an additional test on the basis of a result obtained from the deep learning algorithm.

20. The analysis method of claim 19, wherein
the additional test comprises at least one selected from the group consisting of: a test regarding a coagulation factor; a test regarding a coagulation factor inhibitor; and a re-test regarding a measurement item of measurement performed on the measurement sample.

21. The analysis method of claim 19 or 20, wherein
when there is a plurality of the additional tests, the outputting of the information regarding the additional test comprises outputting a priority ranking of each additional test.

22. The analysis method of any one of claims 19 to 21, further comprising
storing, in association with the data group, a cause, of the prolongation of the blood coagulation time, that has been identified by the additional test.

23. The analysis method of claim 22, further comprising
outputting the associated and stored data group, together with the cause of the prolongation of the blood coagulation time.

24. The analysis method of any one of claims 1 to 23, further comprising:
determining whether or not the blood specimen has prolongation of blood coagulation time, wherein
the outputting of the information regarding the cause of the prolongation of the blood coagulation time is executed when the blood specimen has been determined to have the prolongation, and the outputting of the information regarding the cause of the prolongation of the blood coagulation time is not executed when the blood specimen has been determined not to have the prolongation.

25. The analysis method of any one of claims 1 to 24, further comprising
receiving an output request for the information regarding the cause of the prolongation of the blood coagulation time, wherein
the outputting of the information regarding the cause of the prolongation of the blood coagulation time is executed when the output request has been received.

26. The analysis method of any one of claims 1 to 25, further comprising
on the basis of a kind of a blood coagulation parameter, selecting, from a plurality of deep learning algorithms, the deep learning algorithm to which inputting is performed.

27. The analysis method of any one of claims 1 to 26, wherein
the blood coagulation curve is a curve for obtaining activated partial thromboplastin time or prothrombin time.

28. The analysis method of any one of claims 1 to 27, wherein
the deep learning algorithm has been trained by a data set that includes: the data group obtained from a measurement sample that contains a blood specimen for which a cause for prolongation of blood coagulation time is known and a coagulation time measurement reagent; and a label indicating the cause of the prolongation of the blood coagulation time.

29. The analysis method of any one of claims 1 to 28, wherein
the deep learning algorithm includes a convolution neural network.

30. The analysis method of any one of claims 1 to 29, further comprising:
preparing a measurement sample that contains the blood specimen and a coagulation time measurement reagent; and
generating, from the measurement sample, a plurality of pieces of detection information forming the blood coagulation curve, wherein
the data group is obtained on the basis of the plurality of pieces of detection information that have been generated.

31. The analysis method of any one of claims 1 to 29, wherein
from an analyzer that generates the data group from a measurement sample that contains the blood specimen and a coagulation time measurement reagent, the data group is received via a network, and the received data group is inputted to the deep learning algorithm.

32. An analyzer for a blood specimen, comprising:
a measurement unit configured to prepare a measurement sample that contains the blood specimen and a coagulation time measurement reagent, and configured to output a plurality of pieces of detection information forming a blood coagulation curve, on the basis of the measurement sample; and
a controller, wherein
the controller is configured to
obtain a data group including a plurality of data forming the blood coagulation curve or a differential curve thereof, on the basis of the plurality of pieces of detection information,
input the data group into a deep learning algorithm, and
output, on the basis of a result obtained from the deep learning algorithm, information regarding a cause of prolongation of blood coagulation time of the blood specimen.

33. An analysis program for a blood specimen, the analysis program being configured to cause, when executed by a computer, the computer to execute the steps of:
obtaining a data group including a plurality of data forming a blood coagulation curve or a differential curve thereof;
inputting the data group into a deep learning algorithm; and
outputting, on the basis of a result obtained from the deep learning algorithm, information regarding a cause of prolongation of blood coagulation time of the blood specimen.
